# EUROPEAN PATENT APPLICATION

(11) **EP 4 383 270 A1**
(43) Date of publication of application: **12.06.2024**
(21) Application number: 22212306.9
(22) Date of filing: 08.12.2022
(51) Int. Cl.: G16H 30/40, G16H 50/20, G16H 50/30, G06F 3/0485

(54) **CONTROLLING THE DISPLAY OF MEDICAL IMAGES**

(71) Applicant: Koninklijke Philips N.V., 5656 AG Eindhoven (NL)
(72) Inventor: WIJN, Victor, Eindhoven (NL); SEVENSTER, Merlijn, Eindhoven (NL); VOSBERGEN, Sandra, 5656AG Eindhoven (NL); KNOESTER, Jaap, Eindhoven (NL); BUIL, Vincentius Paulus, Eindhoven (NL); KUHLMAN, Daan, Eindhoven (NL)
(74) Representative: Philips Intellectual Property & Standards

(57) **Abstract**

Presented are concepts for aiding the review of a set of medical images for display at a user interface for viewing by a viewer of the user interface. One such concept includes: obtaining a set of images; determining for each image, via a machine learning method, a measure of confidence that said image contains a representation of one or more predetermined pathologies; defining a minimum time period for which the image is to be displayed, responsive to the measure of confidence for said image; and displaying at least one of the images, wherein for each image displayed it is displayed for at least the minimum time period.

## Description

### FIELD OF THE INVENTION

The present invention relates to the field of medical images and more particularly to the display of medical images for medical image analysis.

### BACKGROUND OF THE INVENTION

It is becoming increasingly common for clinical/medical departments to require the analysis of a wide variety of medical images to correctly assess the condition of an imaged subject/patient. This is particularly important when monitoring or diagnosing for potential pathologies of a subject, e.g., the presence of one or more tumors, polyps, infections or other visible (but internal) abnormalities.

It is recognized there is therefore an increasing demand for imaging examinations, which is growing exponentially every year. Studies predict a 7% growth in demand over the coming 5 years. Additionally, technological advancements that improve diagnostic capabilities may also increase workload and complexity. This combines to have a significant effect on clinicians, with the profession showing increased burnout rates and efflux among medical image analysts (e.g., radiologists).

One potential source of relief for this pressure is to increase the use of supporting artificial intelligence (AI) technologies. However, the current use of many AI applications is limited. If human-AI collaboration is improved, AI solutions may prove truly capable of supporting effective image interpretation and a positive staff experience.

In general, when analyzing a set or sequence of medical images (e.g., a 3D volume defined by a stack of 2D image slices or a temporal sequence of medical images), an image analyst will often move or scroll through the set or sequence of images in order to identify any potential pathologies.

However, there is an ongoing risk that the analyst will overlook or miss a potential pathology, which could lead to negative repercussions on the future health of the subject of the set of sequence of medical images.

### SUMMARY OF THE INVENTION

The invention is defined by the independent claims. Dependent claims represent beneficial embodiments.

According to examples in accordance with an aspect of the invention, there is provided a computer-implemented method for aiding the review of a set of medical images for display at a user interface for viewing by a viewer of the user interface, the computer-implemented method comprising: obtaining the set of medical images; processing the set of medical images using at least one machine-learning method, to predict, for each medical image, a measure of confidence that said medical image contains a representation of one or more predetermined pathologies; defining, for each medical image in the set of medical images, a minimum time period for which the medical image is to be displayed, when displayed at the user interface, responsive to the measure of confidence for said medical image; and displaying at least one of the medical images in the set of medical images at the user interface, wherein, for each medical image displayed, the time period for which said medical image is displayed is no less than the defined minimum time period for that medical image.

The present disclosure provides a technique for controlling how long a medical image is displayed on a screen responsive to a confidence that the medical image contains a representation of a pathology or pathologies.

Proposed concepts thus aim to provide schemes, solutions, concepts, designs, methods and systems pertaining to aiding and/or improving the display of medical images for the purpose of medical image analysis.

As is known in the art, reviewing medical images of e.g., Computed Tomography (CT) images involves review of stack of images comprising of slices of an anatomy. Reviewing stack of images in radiology imaging is more time consuming than reviewing a single 2-dimensional image, such as chest X-ray. During the review the radiologist must scroll through the full series to get an idea of the image and identify specific parts where there might be findings, i.e., particularly relevant anatomical features. When coming across findings, the radiologist often manually scrolls through findings to get a good view on the finding. During this process, they may scroll through certain parts of the image quicker or slower, depending on what is normal or abnormal. Sometimes, as is known in the art, this variance could lead to missing certain findings or the reviewing process being more time consuming.

Herein is proposed a system and a method that automatically adapts the scrolling speed depending on the confidence score of AI findings in the slices that are scrolled through. AI findings are determinations made by the AI as to relevant features, comprising a determination of whether or not said medical image contains a representation of one or more predetermined pathologies.

A high scrolling speed may mean that images are displayed for a shorter time period, and a low scrolling speed may mean that images are displayed for a longer time period. Accordingly, scrolling speed and the time period for which an image is displayed are directly linked, in an inverse proportional relationship. Thus, if an individual is permitted to control the scrolling through a set or sequence of medical image, references herein to scrolling speed or time period for which an image is displayed relate to the same feature, since they are the inverse of each other.

During an image review, a radiologist scrolls through the anatomy of a patient, looking for irregularities or abnormalities. The radiologists explore findings that are related to the patient's health problem and suspected diagnosis, pathologies, etc. Therefore, they must investigate the whole scanned area, which can be quite cumbersome. These images are built up from a slice, or multiple slices that are made by the scanner (CT, PET-CT, MRI, X-ray, ULDCT, ultrasound, digital pathology, etc.). These slices allow for a multidimensional view through specific parts of the patient's body. In some embodiments, the set or sequence of images / slices represent a consecutive sequence of adjacent layers of the anatomy.

Artificial Intelligence (AI) is applied for assisting the radiologist to determine findings that may be interesting to look at. However, there is a number of disadvantages associated with this, e.g., cumbersome review of AI recommendations.

In the present invention, the information from the AI is leveraged to optimize scrolling speed, to enhance the overall reviewing performance and user experience of the radiologist. For example, the invention may be employed in an imaging review system when scrolling speed is adaptive to AI findings and the AI measure of confidence. The mechanism to advance images may be based on scrolling with a mouse wheel and the system adaptively slows down the scrolling speed in the system, according to the measure of confidence, as determined by the AI. Where confidence is low, images may be displayed for longer (i.e., scrolling speed may dynamically decrease), to allow the operator additional time to review the image.

The present disclosure provides a technique for controlling how long a medical image is displayed on a screen responsive to a confidence that the medical image contains a representation of a pathology or pathologies. This approach can, for instance, be used to control a "scrolling speed" of an individual as they move through the images, or to control a speed at which images are successively displayed (e.g., by a video). In this way, the speed at which it is possible to move to display a next image in the set or sequence is dependent upon the confidence that the currently displayed medical image contains (or does not contain) a pathology or pathologies.

The proposed approach assists a viewer of the medical images to review and/or focus upon the most potentially relevant medical images, e.g., those images for which a machine-learning method is not confident whether or not it contains the pathology/pathologies. This approach reduces a likelihood that a potential pathology will be missed or overlooked by a viewer of the set or sequence of medical images.

The advantage of the present invention results in an advantage of improved reviewing capability for a clinician that would not be possible without adopting the approach herein claimed. The invention in enhances the workflow of image reviews for radiologists, and allows for efficient guided image reviewing, particularly when cognitive performance is low. The invention allows time to be saved in an image review. It would save time in an image review, and support staff in their work resulting in improved staff experience. The invention reduces the chance of diagnostic inaccuracies and missed finding.

The present invention recognizes that a significant cause of an image analyst or radiologist missing or overlooking a pathology is due to high speeds of moving through images in a set or sequence, e.g., high scrolling speeds. The proposed approach mitigates or overcomes this problem by controlling the maximum possible speed of moving through the image(s) based on the confidence level that a machine-learning method has identified a pathology within the images.

Each medical image in the set or sequence of 2D image may be, for instance: at least one image slice of a 3D medical image visualization; an image in a temporal sequence of medical images (stack of images); or a part of a larger medical image. The sequence may comprise a series of slices representing adjacent layers of the anatomy, and/or may comprise a series of slices of which at least one adjacent pair in the sequence does not represent adjacent layers of the anatomy. That is, the sequence of images may represent discontinuous layers of anatomy, may represent continuous, adjacent layers of anatomy, or may represent a combination of continuous and discontinuous layers of anatomy.

This invention may be employed in image reviewing software or in advanced visualization software.

The invention provides the ability to review radiology cases more effectively by improving human-centered AI interaction. By analyzing and mapping the AI findings in the exact 3D location within the anatomy, a spatial-aware scrolling speeds can be derived for this specific review. This results in a system that adaptively adjusts scrolling speeds for every active viewing angle and position within the anatomy. This way, review behavior of the radiologist is supported and enhanced based on the content-aware AI findings of the radiology image. This allows for lowering cognitive load and reading time, while improving user experience of the radiologist.

In embodiments, for each medical image, the minimum time period is configured to increase responsive to a measure of confidence for that medical image decreasing.

In this way, a viewer of the medical image(-s) is given longer time period to view a medical image if the machine-learning method is less confident that the medical image contains (or does not contain) a pathology, i.e., when the measure of confidence is low. The current approach reduces a likelihood that a pathology will be missed by both the machine-learning method and the viewer of the medical image(s).

In embodiments, the step of processing the set of medical images using the at least one machine-learning method further produces, for each medical image, a pathology indicator that indicates a prediction of whether or not said medical image contains the representation of the one or more predetermined pathologies, and the step of defining, for each medical image, the minimum time period is further responsive to the pathology indicator for said medical image.

In embodiments, for each medical image, the minimum time period is configured to increase responsive to the pathology indicator for said medical image indicating that said medical image is predicted to contain a representation of the one or more predetermined pathologies. For example, one or more predetermined pathologies may be defined, and when the AI determines that one of these one or more predetermined pathologies is present, the minimum time period for an image may be increased compared to where none of the one or more predetermined pathologies is determined to be present in an image.

This approach will increase the time spent by a viewer in reviewing/viewing the medical image(s) determined by the AI to contain one or more particular pathologies. This is advantageous as the viewer/analyst has the ultimate responsibility to thoroughly check all potential pathologies (e.g., to assess their condition, size, localization). It is therefore important when coming across a pathology for the first time, that the viewer is given sufficient time to check the pathology.

In embodiments, the step of displaying at least one of the medical images in the set comprises: displaying, via the user interface, a first medical image of the set of medical images; and responsive to receiving a scroll input from an input user interface, moving to a neighboring medical image, to the first medical image, in the set of medical images only if and/or when the first medical image has been displayed, via the user interface, for at least the minimum time period for the first medical image.

In this way, the viewer may be able to effectively scroll through the set or sequence of images, wherein the scrolling speed (e.g., the speed at which the individual can move to a next image in the set or sequence) is dependent upon the confidence that the current medical image contains (or does not contain) a pathology or pathologies.

In embodiments, the step of defining, for each medical image, the minimum time period is further responsive to the measure of confidence of at least one neighboring medical image, in the set of medical images, to the said medical image.

In embodiments, the method further comprises: obtaining, for each medical image, a review indicator that indicates whether or not the medical image, or any potential pathology in the medical image, has been previously reviewed by a clinician, wherein, for each medical image, the minimum time period for said medical image is further responsive to the review indicator of the medical image.

In embodiments, for each medical image, the minimum time period decreases responsive to the review indicator indicating that the medical image, or any potential pathology in the medical image, has been previously reviewed by a clinician.

In embodiments, the method further comprises obtaining a measure of concentration of the viewer of the user interface, wherein, for each medical image, the minimum time period for said medical image is further responsive to the measure of concentration. In a further embodiment, for each medical image, the minimum time period is configured to increase responsive to the measure of concentration decreasing.

It has been recognized that reduced levels of concentration will cause a clinician to miss or overlook pathologies within a set or sequence of images. By increasing the minimum time period for each medical image responsive to the level of concentration, the chance that a pathology will be missed is further reduced.

In embodiments, the method further comprises obtaining an experience level of the viewer of the user interface, wherein, for each medical image, the minimum time period for said medical image is further responsive to the experience level. In some embodiments, the method could also be made dependent on the imaging indication (e.g., if a pathology is present), the nature of the detected finding (e.g., malignancy) and whether the finding was detected in a prior examination.

In embodiments, for each medical image, the minimum time period is configured to decrease responsive to the experience level of the viewer increasing.

Experience level may mean a time at a particular professional grade, an experience within the profession, an experience level with the equipment and/or AI algorithm, an experience level in terms of number of relevant images analyzed or relevant diagnosis performed, or any experience level as defined by the medical establishment or a relevant body, or any combination of these parameters.

In embodiments, the method further comprises, whilst displaying the at least one of the medical images, providing haptic feedback to the viewer of the user interface responsive to the minimum time of the displayed medical image.

Haptic feedback may comprise, for example, increased scroll resistance on a mouse wheel or on a dial or other device used to scroll the images. Other feedback types may be possible depending on the need.

According to another aspect of the invention, there is provided a computer program product comprising computer program code means which, when executed on a computing device having a processing system, cause the processing system to perform all of the steps of any herein described method.

According to another aspect of the invention, there is provided a processing system for aiding the review of a set of medical images for display at a user interface for viewing by a viewer of the user interface, the processing system being configured to: obtain the set of medical images; process the set of medical images using at least one machine-learning method, to predict, for each medical image, a measure of confidence that said medical image contains a representation of one or more predetermined pathologies; define, for each medical image in the set of medical images, a minimum time period for which the medical image is to be displayed, when displayed at the user interface, responsive to the measure of confidence for said medical image; and display at least one of the medical images in the set of medical images at the user interface, wherein, for each medical image displayed, the time period for which said medical image is displayed is no less than the defined minimum time period for that medical image.

In an embodiment, the minimum time period per image determination may be responsive to the measure of confidence of an adjacent or nearby image. In some embodiments, this may be determined based on propagation of images.

Accordingly, the minimum time period per image can be prevented from changing abruptly between adjacent images. The invention may thus comprise an adaptive scrolling mechanism that prevents the scrolling speed suddenly changing between adjacent images. This feature may comprise regulating the rate of change of the scrolling speed when moving through the slices. If a series of adjacent slices abruptly transition from "not-confident" to "confident", the scrolling speed can be transitioned smoothly from slow to fast scrolling for series of adjacent images, and if the series of adjacent slices abruptly transition from "confident" to "not confident", the scrolling speed can be transitioned smoothly from fast to slow scrolling for a series of adjacent images. This way, it is made sure that the user can be aware of upcoming attentiveness of what is shown on the screen, and user experience is improved.

According to further examples in accordance with an aspect of the invention, there is provided a computer-implemented method for aiding the review of a set of medical images for display at a user interface for viewing by a viewer of the user interface, the computer-implemented method comprising: obtaining the set of medical images; processing the set of medical images using at least one machine-learning method, to determine or predict, for each medical image, a predetermined pathology characteristic; defining, for each medical image in the set of medical images, a minimum time period for which the medical image is to be displayed, when displayed at the user interface, responsive to the predetermined pathology characteristic; and displaying at least one of the medical images in the set of medical images at the user interface, wherein, for each medical image displayed, the time period for which said medical image is displayed is no less than the defined minimum time period for that medical image. The predetermined pathology characteristic may comprise one or more of: a level of confidence that said medical image contains a representation of one or more predetermined pathologies; the nature of the pathologies; the size and/or number of the pathologies; the indication of the study; or any combination of the above.

In the embodiments, the concept is adapted responsive to the state of the person interacting with the system.

In embodiments, the system can analyze if findings have been reviewed sufficiently.

The present application has one or more technical benefits.

One of the advantages may be to enhance the workflow of image reviews for radiologists.

Another advantage may be to allow for efficient guided image reviewing of diagnostic images.

Another advantage may be more efficient use of the equipment by optimizing the time spent for an image review. Furthermore, better staff experience may be achieved.

Another advantage may be in reducing the chances of diagnostic inaccuracies and/or missed findings in review of diagnostic images.

Another advantage may be in improved Quality Assurance/Quality Control (QC/QA) as the system of the present invention may analyze if findings have been sufficiently reviewed.

Other advantages may be possible within the context of the present application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig 1 is a simplified block diagram of a proposed embodiment of a system for aiding the review of a set or sequence of medical images for display at a user interface for viewing by a viewer of the user interface;
Fig 2 is a simplified flow diagram of a method for aiding the review of a set or sequence of medical images for display at a user interface for viewing by a viewer of the user interface;
Fig 3 is a chart showing a variation of minimum time period / scrolling speed against confidence level, according to an embodiment;
Fig 4 is a chart showing a variation of minimum time period / scrolling speed against confidence level, according to another embodiment; and
Fig 5 is a simplified block diagram of a computer within which one or more parts of an embodiment may be employed.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figs are merely schematic and are not drawn to scale. It should also be understood that the same reference numerals are used throughout the Figs to indicate the same or similar parts.

In the field of radiology there is a high level of burnout and therefore the need for supporting radiologists through technology such as AI is rapidly rising. However, the findings made by AI may need to be checked carefully, particularly where the technology is new or confidence in the technology is not yet mature.

Radiologists often perform a "quick scan" of three-dimensional image volumes or through specific sets or sequences within an imaging exam. Radiologists perform these quick scans to get a "Gestalt" of the image, i.e., a sense of the disease state of the patient that guides the remainder of the interpretation. While performing a quick scan through an imaging set or sequence or part thereof, there is a risk that radiologists miss a potentially relevant finding if they scroll too fast. This could lead to adverse health outcomes of the patient; in case the missed finding is clinically relevant and is not treated appropriately. For instance, if a radiologist quickly scrolls through the lungs and misses a small lung nodule, the nodule might grow and metastasize if it is malignant.

The risk of missing clinically relevant findings is greater if such findings are "incidental", i.e., do not generate symptoms triggering the radiological examination. For instance, if the patient has a cardiac condition, the radiologist will inspect the cardiac region closely and might miss findings unrelated to the patient's cardiac condition. This is called the satisfaction of search error in the literature. Also, there is an increased risk of missing a finding when radiologists are distracted, obstructed from their preferred way of working, or when they are tired.

Hence, a solution is needed that facilitates an enhanced workflow and user experience for human-AI interaction in radiology imaging without losing review quality.

The present disclosure provides a technique for controlling how long a medical image is displayed on a screen responsive to a confidence that the medical image contains a representation of a pathology or pathologies. This reduces a likelihood clinically relevant findings being missed or overlooked, and reduces the cognitive load on radiologists. Furthermore, the quality of care is improved since a more accurate diagnosis may be made since there is a reduced likelihood of relevant features being missed.

According to the invention, diagnostic AI can be used to perform a pre-read of the examination and its findings can be used to guide the radiologist during scans in a minimally obtrusive manner, i.e., by adapting down (reducing) the scrolling speed based on the finding density and corresponding AI-confidence. Therefore, this invention proposes a system or method that allows optimized scrolling behavior in image review, considering the necessary review engagement, detail, and thoroughness, based on the confidence measures of AI findings that are established in relation to the anatomy.

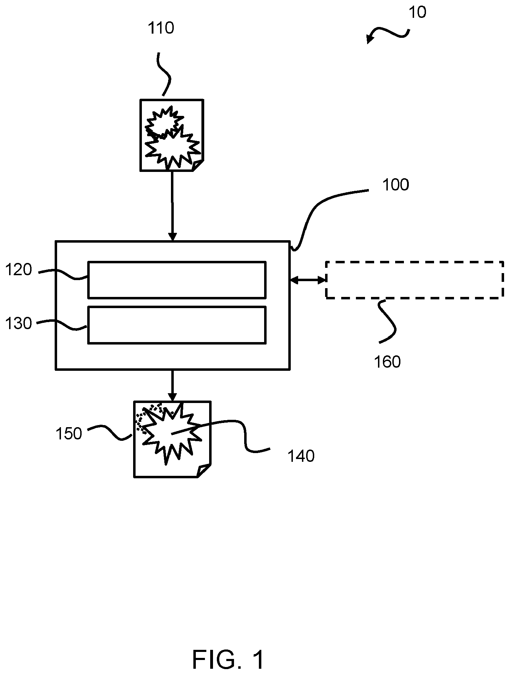

Fig 1 is a simplified block diagram of a proposed embodiment of a system 10 for aiding the review of a set or sequence of medical images for display at a user interface 150 for viewing by a viewer of the user interface.

A system 10 according to a proposed embodiment is configured to obtain via a controller 100 (e.g., receive via an input interface of the controller 100) a set or sequence of medical images 110, and to display at least one image 140 of the images 110 at a user interface 150 (such as a screen). The system 10 is configured to analyze, via an analysis component 120, each medical image in the set or sequence 110 and use machine-learning and/or some form of AI to predict for each image a measure of confidence that the image contains a representation of one or more pathologies. The analysis component 120 also determines a corresponding minimum time period that each image should be displayed, according to the measure of confidence determined for each of the images. When images are displayed on the user interface, the system 10, via a display component 130, ensures that the image is displayed on the user interface 150 for at least the minimum time period. Throughout this disclosure, an image for which a minimum time period has been determined may be referred to as a minimum time period image.

Accordingly, when a minimum time period image is be displayed, the display component 130 ensures that the image is displayed for at least the minimum time period, which may be regardless of any user input or request to move to another image. Any user input may simply be ignored during the remained of the time period, or may be cached so the user input is responded to once the remaining time period has elapsed. Feedback may be provided to the operator in this instance, to re-assure the operator that the device has not "frozen", but instead is operating deliberately to prevent the current image from being changed.

The system 10 may comprise one or more optional input device 160 (such as a keyboard and mouse), that is connected to the controller 100, and which can be used to control what is displayed on the screen. Alternatively, any feature of the optional input device 160 may be implemented directly via the user interface (for example, via a touch screen display which may include virtual or physical controls). The optional input device 160 and user display 150 may comprise similar or identical controls, providing increased flexibility.

The system 10 may comprise means for an operator to advance or scroll through the images 110 in set or sequence. For example, according to a "back" and "forward" key presses on virtual or physical keys, images may be projected on the user interface sequentially, in a forward or reverse order or sequence respectively. The system may allow the user to scroll through the set or sequence of images 110 in sequence, wherein the speed at which they are displayed is according to a movement of a virtual or physical scroll wheel (sometimes called a "jog wheel"), or a of virtual or physical linear slider (sometimes called a "seek bar"), or of alternative advancement means, determines the image that is displayed. For example, the image that is displayed may accord directly to the rotation of a scroll wheel or the position that the linear slider is moved to, such that when the wheel or slider is no longer moved the image displayed is not changed until a further input is received; as the wheel is rotated or the slider moved the displayed image varies according to the position of the wheel or slider. Alternatively, the movement of the virtual of physical scroll wheel or linear slider or other advancement means may be used to control the rate at which images are advanced, i.e., such that the rate of advancement is maintained for so long as the scroll wheel, linear slider or other advancement means is not adjusted, or for so long as a further input, such as a key press corresponding to a 'stop' command is not received. For example, rolling the mouse wheel "one click" may advance the images as a first rate, rolling the mouse wheel "two clicks" may advance the images at a faster rate, and moving the mouse wheel back "two clicks" may stop the images advancing until the mouse wheel is moved again. Moving the mouse wheel in the opposite direction may cause the order in which the images are displayed to be reversed, at a rate according to how far the mouse wheel is moved.

The system 10 may allow the images 110 to be displayed in order, according to a predetermined time period, in the form of a "slide show", and controls may be provided to allow the rate and/or time period to be adjusted. For example, a "speed decrement" and a "speed increment" button may respectively decrease or increase the rate at which images are displayed.

Alternatively, or in addition, inputs may allow the advancement rate of images within the slideshow to be specified, for example, a rate of one image per second, two images per second, or one image every two seconds. The system may allow images to be tagged or flagged via user input, and the system may be configured to allow tagged images to be displayed on demand (without also showing untagged images). Tagging may, for example, allow an operator to flag that: an image has been reviewed; an AI finding has been reviewed; an AI finding is agreed with; an AI finding is not agreed with; the image needs further review (perhaps a second professional opinion).

Due to the operation of the analysis component 120 and display component 130, the system may affect the display of an image 140 on the user interface 150. For example, the analysis component 120 may determine that the fifth image in the set or sequence of images 110 may comprise one or more determined pathologies, and provide an accordingly provide a measure of confidence (for example, "high"). Methods and apparatus for the determination of a measure of confidence that an image comprises a determined pathology are known in the field, for example as disclosed in the European Patent Application having publication number EP3654278A1. Any such known method or apparatus may be employed to determine a measure of confidence in the subject invention. The measure of confidence may be expressed, as a non-limiting example, by a number on a scale from a scale minimum (for example 0) to a scale maximum (for example 10). For example, the measure of confidence determined in relation to the fifth image may correspond to a number 7 on a scale of 0 to 10, which may mean high likelihood that a determined pathology is present in the image. Accordingly, the analysis component 120 may determine a minimum time period, for example 10 seconds, according to the measure of confidence - and the display component 130 may ensure that that image, when displayed, remains on the screen for at least the minimum time period.

According to the preceding example, an operator may manually advance the image(s) 140 displayed on the screen to scroll, rotate or move through the images in order or sequence. However, once the fifth image has been displayed, the display component may prevent the image from being changed until that fifth image has been displayed e.g., for at least 10 seconds, which corresponds the time period according to the measure of confidence. This may ensure that the operator, who may be fatigued, does not miss an important feature of one or more images, since they are unable to move away from the image until the minimum time period has elapsed. Feedback may be provided to the operator to further prompt them that this image may require particular attention.

In an embodiment, this feedback may be provided to the operator when an image 140 having a minimum time period is displayed, and optional components (such as a speaker) may be present to provide this feedback. For example, when such a minimum time period image is displayed, one or more sensory feedback (i.e., user-perceptible indication) may be provided, such as a visual indication, an aural (sound) indication or a haptic indication. For example, when a minimum time period image is displayed, one or more of the following may happen: the image may momentarily flash on the screen, a countdown in relation to remaining time of the associated time period may be displayed, a sound may be made, haptic feedback may be provided. This feedback alerts the operator that particular attention may be required. Visual means for alerting the operator may comprise adjusting the color of the image (for example, providing a different background and/or foreground color), or a visual indication such as notification may be provided on the user interface 150, or the image may otherwise be manipulated. For example, the image may be momentarily enlarged and then shrunk back to its original size to alert the operator that a minimum time period image is being displayed.

This feedback may be provided when an image is displayed, for example immediately or after a pause (which may vary according to the time period), and/or may be provided only when the operator provides an input to move to another image in the set or sequence 110. For example, when a minimum time period image is displayed, no feedback may be provided so long as the image is maintained on the user interface for the minimum time period - for example, because the operator has noticed a feature that they wish to observe or study. However, if the operator then attempts to adjust the image on the screen before the minimum time period has expired, feedback may then be provided, alerting the operator that a minimum time period image has been displayed and that the minimum time period has not elapsed. Accordingly, feedback may be more selectively provided, and may prompt the operator to consider the image in more detail than they initially intended to, as a result of the operator attempting to change the image.

In an embodiment, the system may be configured to allow the operator to define when feedback is provided and what feedback is provided. Thus, the feedback system may be entirely configurable according to operator needs.

In an embodiment, the system may be configured to allow the operator to override a time period, for example where they have thoroughly observed and image and determined that the image has been incorrectly analyzed by the machine learning. Optionally, the potentially incorrectly analyzed image may be flagged, and this information may be provided back to the analysis component 120 or to a supervisor or technician, so that the image machine learning may be improved.

In an embodiment, haptic feedback may optionally be provided, via one or more of the optional input device 160, the user interface 150, or other means. Haptic feedback may be provided for a specific image in accordance with the minimum time period of the displayed image.

In an embodiment, the analysis component 120 provides determined a minimum time period that is configured to increase responsive to a measure of confidence decreasing. The minimum time period may be inversely correlated with the measure of confidence. For example, a first image with a relatively higher measure of confidence may be displayed for a longer period of time than a second image with a relatively lower measure of confidence.

In an embodiment, the analysis component 120 is configured so that: when processing the set or sequence of medical image, it produces, for each medical image, a pathology indicator that indicates a prediction of whether or not said medical image contains the representation of the one or more predetermined pathologies; and when defining, for each medical image, the minimum time period, it is further responsive to the pathology indicator for said medical image. For example, when a particular pathology is encountered, the analysis component may generate a pathology indicator which affects the minimum time period determined for that image.

In some embodiments, the analysis component 120 is configured to increase the minimum time period responsive to the pathology indicator for said medical image indicating that said medical image is predicted to contain a representation of the one or more predetermined pathologies. For example, the analysis component 120 may increase the minimum time period for an image when a particular pathology indicator is determined for an image.

In some embodiments, when a minimum time period image is displayed the display component 130 maintains the image on the user interface 150 for the minimum time period and the controller 100 may disregard any user input, such as an input from a scroll wheel, whilst the image is displayed. The input may be entirely disregarded or may be cached so that the instructions may be implemented after the remainder of the minimum time period. If the minimum time period image is displayed as part of a predetermined order or sequence (such as an automatic slideshow with automatic advancement to the next image after a fixed period), the order or sequence may be paused until the minimum time period, after which the order or sequence may be continued according to the fixed period.

In some embodiments, the analysis component 130 determines the minimum time period for an image comprising a pathology in consideration of the measure of confidence of at least one neighboring image. For example, the analysis component 130 may increase or decrease the minimum time period of an image with a particular measure of confidence, due to the determined measure of confidence of a neighboring image.

In some embodiments, the analysis component 120 obtains a review indicator that indicates that the image has previously been reviewed by an operator and adapts the minimum time period according to the presence of this indicator. For example, the analysis component may reduce the minimum time period for an image, where the analysis component 120 receives a review indicator that confirms that an image an image has previously been reviewed by an operator.

In an embodiment, the controller 100 determines a measure of concentration of a viewer of the user interface 150. For example, the controller 100 may use optical sensors to determine the alertness of a viewer, according to body positioning and facial cues such as eye movement or the movement of eyelids. Approaches for monitoring a concentration level are set out, inter alia, by US Patent Applications having publication numbers US 2011/037,850 A1 and US44929599A. According to this measured concentration, the analysis component 120 adjusts the minimum time period. This may include providing a minimum time period for every image in the set or sequence of images 150 or increasing the minimum time period by a fixed or proportional amount but only for minimum time period images. For example, where the controller 100 determines that an operator is of low alertness the minimum time period associated with each minimum time period image may be increased by 10 seconds. The determination of alertness may comprise analyzing one or more factors and determining if a predetermined threshold has been exceeded. For example, multiple alertness states may be determined: one or more (but less than three) eye lid close in a minute may be considered 'normal alertness'; three or more (but less than six) may be considered 'lower alertness'; and six or more may be considered 'very low alertness'. As an example: when a first state (e.g. 'normal alertness') is determined, the minimum time period may be varied by a first variation (e.g., may be unchanged), when a second state (e.g., 'lower alertness') is determined, a second variation may be made to the minimum time period of each minimum time period image (e.g. an increase of five seconds); and when a third state (e.g., 'very low alertness') is determined, a second variation may be made to the minimum time period of each minimum time period image.

In an embodiment, the analysis component 120 determines a level of experience of the viewer of the user interface 150 and adapts the minimum time period for each medical image. For example, if the user is determined to be an operator of a particular seniority (e.g., a junior operator), which may be determined by a user account logon or through an authentication mechanism such as a key card with an encoded experience ranking, then the minimum time period for each image may be increased according to the determined seniority. Seniority may comprise a determined grade in an organizational structure, a specific role, or another defined experience ranking, such as number of relevant medical or clinical procedures undertaken, or number of years worked at a relevant grade. In an embodiment, the analysis component 120 determines whether the level of experience is above a threshold, and if it is then the minimum time period is decreased accordingly. For example, the minimum time period may be decreased by a fixed percentage, e.g., 10%, 30% or 50%, of the minimum time period. Alternatively, the minimum time period per image may be reduce by a fixed number of seconds if the threshold is exceeded. In an embodiment, a plurality of time period reductions according to a plurality of experience thresholds is provided. In an embodiment, the experience level(s) and associated reduction(s) in the minimum time period are predetermined. In another embodiment, the user or an administrator can configure the experience level(s) and associated reduction(s) in the minimum time periods.

In an embodiment, the analysis component 120 adapts the minimum time period per image determination according to the measure of confidence of an adjacent or nearby image. For example, the analysis component 120 may, when determining a minimum time period for a current image in the set or sequence, determine the measure of confidence of a next image in the set of sequence, and adapt the minimum time period of the current image accordingly. For example, if the measure of confidence associated with the next image is relatively lower, the time period for the current image may be increased relative to when the measure of confidence associated with the next image is relatively higher. The minimum time period per image determination may be adapted according to the measure of confidence of more than one adjacent or nearby images, for example: the next two or three images; the preceding two or three images; or the preceding two and succeeding two images; or the preceding image and the next image.

In an embodiment, the analysis component 120 is configured to: process the set of medical images using at least one machine-learning method, to determine or predict, for each medical image, a predetermined pathology characteristic; and define, for each medical image in the set of medical images, a minimum time period for which the medical image is to be displayed, when displayed at the user interface, responsive to the predetermined pathology characteristic. The predetermined pathology characteristic may comprise one or more of: a level of confidence that said medical image contains a representation of one or more predetermined pathologies; the nature of the pathologies; the size and/or number of the pathologies; the indication of the study; or any combination of the above.

The variations on the embodiment set out herein may be combined individually with the embodiment 10 or may combined in any combination within the embodiment 10. For example, all variations on the embodiment may be combined in one exemplary embodiment according to the invention.

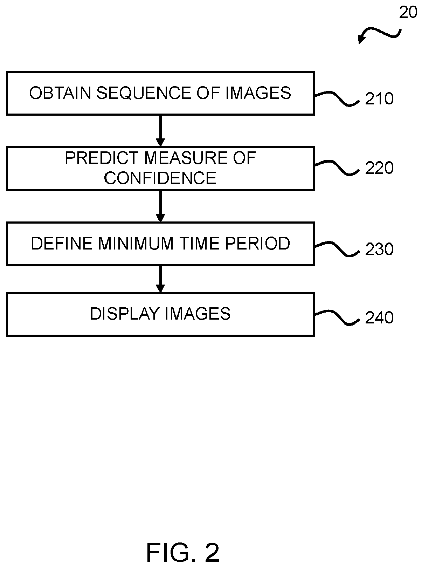

By way of further illustration and description, an exemplary method according to an embodiment will now be described with reference to Fig 2.

Fig 2 is a simplified flow diagram of a method 20 for aiding the review of a set or sequence of medical images for display at a user interface for viewing by a viewer of the user interface.

The method 20 comprises a first step 210 of obtaining a set or sequence of images. This step may comprise receiving a scanned anatomy image package in slices (i.e., sequence of images), and may, for example, comprise importing or receiving a DICOM format file comprising this information. A second step 220 comprises processing the set or sequence of images to predict, for each image, a measure of confidence that said image contains a representation of one or more predetermined pathologies. Any known technique for processing images to determine if one or more predetermined pathologies is present, with an associated level of confidence, may be used.

Second step 220 may comprise running an AI abnormality algorithm (e.g., lung nodule CAD, lymph node CAD, bone fracture CAD, pancreatic cancer CAD) through which confidence scores may be generated. As one exemplary method, the algorithm may attempt to find representations of one or more of predetermined pathologies within a slice (i.e., an image in the set or sequence of images) and establish a measure of confidence in relation to any finding of one or more predetermined pathologies. Optionally, the algorithm may determine a measure of confidence per finding within the anatomy (volumetric), i.e., consider a plurality of adjacent images in the set or sequence of images to determine the presence of a one or more predetermined pathologies within a particular image in the set or sequence. The algorithm may then determine a confidence score per slice based on findings. In a variation, this confidence score may be based on the lowest measure of confidence in the slice (in the case of multiple findings in a slice).

In Second step 220, the AI findings are defined (with certain confidence level) and linked to the content of the radiology image. Additionally, areas (collections of image slices) without AI findings may be defined (with a certain confidence level) and linked to the content of the radiology image. The definition of the AI findings is about the location, orientation, dimensions, and the connection to the content within the anatomy. The areas in the image without AI findings, are considered and defined as slices without finding.

A third step 230 comprises defining a minimum time period per image according to the determined measure of confidence. Where there are multiple AI findings for an image in the set or sequence, the minimum time period may increase according to the number of findings and may increase linearly with that number (for example, an image with three AI findings may be displayed for three times as long as an image with one AI finding).

The third step 230 of defining a minimum time period per image according to the determined measure of confidence, may comprise combining the data on: (1) slices with and without findings and the corresponding confidence levels, and (2) the current review state regarding the slice "currently in review" and its surrounding slices. Based on these confidence scores and the review location within the anatomy, minimum time period per image (i.e., a current scrolling speed) can be determined. This minimum time per image may be set to be less (i.e., scrolling speed will be faster) when the AI is extremely confident about its finding or non-finding. The minimum time period per image may be set to longer (i.e., scrolling speed will be slower) when the AI is not confident about its results (i.e., when the measure of confidence is low).

The third step 230 of defining a minimum time period per image according to the determined measure of confidence, may comprise an adaptive scrolling mechanism, so that the scrolling speed may not suddenly change between adjacent images. This element comprises regulating the scrolling speed when moving through the slices. If the slices abruptly transition from "not-confident" to "confident", the scrolling speed user should transition smoothly from slow to fast scrolling. This way, it is made sure that the user can be aware of upcoming attentiveness of what is shown on the screen. Accordingly, the minimum time period per image determination may be responsive to the measure of confidence of an adjacent or nearby image, so that the minimum time period per image does not change abruptly between adjacent images. Alternatively, once a minimum time period per image has been determined, a smoothing algorithm may be applied, to lessen harsh variations between adjacent or nearby images, i.e., to smooth (reduce) the variation.

A fourth step 240 comprises displaying an image from the set or sequence of images, at the user interface, for no less than the minimum time period defined for that image (as defined in step 230). In this step, the currently viewed slide may be known based on the image that is being projected. Alternatively, a plurality of adjacent images may be simultaneously provided on the user interface, one of which may be a "main" image that may be larger than the rest. In this case of multiple images being provided, the actively viewed image can be established through processing the mouse-hovering location, but also through eye-gaze behavior as an advanced embodiment. The current image slice that is being looked at can be determined via the corresponding slice number. Accordingly, where multiple images are provided on the user interface at once, once the actively viewed image has been determined, that image can be retained on the display for no less than the minimum time period defined for that image.

In a variation of the method 20, the third step 230 comprises increasing the minimum time period responsive to a measure of confidence for that medical image decreasing.

In a variation of the method 20, the second step 220 comprises producing, for each medical image, a pathology indicator that indicates a prediction of whether or not said medical image contains the representation of the one or more predetermined pathologies, and the third step 230 comprises adapting the minimum time period for a said image responsive to the pathology indicator for said medical image. In a further variation of the method 20, the third step 230 comprises decreasing the minimum time period is configured to decrease responsive to the pathology indicator.

In a variation of the method 20, the third step 230 comprises determining a level of alertness of an operator and varying the minimum time period for an image or for every image accordingly.

In a variation of the method 20, the fourth step 240 comprises displaying, via the user interface, a first medical image of the set or sequence of medical images, and, responsive to receiving a scroll input from an input user interface, moving to a neighboring medical image, to the first medical image, in the set or sequence of medical images only if and/or when the first medical image has been displayed, via the user interface, for at least the minimum time period for the first medical image.

In a variation of the method 20, the third step 220 comprises obtaining, for each medical image, a review indicator that indicates whether or not the medical image, or any potential pathology in the medical image, has been previously reviewed by a clinician, and, for each medical image, the minimum time period for said medical image is further responsive to the review indicator of the medical image.

In a variation of the method 20, the third step 230 comprises determining a measure of concentration of an operator and varying the minimum time period for an image or for every image accordingly. In a further variation, the minimum time period is increased responsive to the measure of concentration decreasing.

In a variation of the method 20, the third step 230 comprises obtaining an experience level of the viewer of the user interface, and, for each medical image, adjusting the minimum time period for said medical image responsive to the experience level.

In a variation of the method 20, the fourth step 240 comprises providing haptic feedback according to the minimum time period.

In a variation of the method 20, the second step 220 comprises an image analysis AI to scan and analyze the entire radiology image volume of the anatomy. Therefore, it determines whether there is an AI finding in specific slices, or not. Additionally, the positioning, dimensions, orientation are established and connected to the anatomy of the body in that slice. This way, a 3-dimensional mapping of findings (and non-findings) in the body is established that can be shown in a viewing window on the user interface. Additionally, the measure of confidence of findings and non-findings are determined and connected to that slice. The 3-dimensional mapping of findings and non-findings of the body is extended with these measures of confidence. During an image review, the radiologist might set different slice thicknesses to optimize the readability of an image. If the slice thickness is adjusted, the scores and indications are averaged over the considered separate slices that were originally assessed.

In a variation to the method 20, the third step 230 comprises applying one of a plurality of predefined minimum time periods to an image according to the determined measure of confidence, in a number of adjacent bandings. For example, a plurality of confidence levels may be determined, each of which is associated with a discrete minimum time period, and the minimum time period associated with a range of the measure of confidence may decrease as the measure of confidence increases. As an example, with reference to the table below, at one end of a range, a longer minimum time period (relating to slow scrolling speed) may be applied to images where there is a low level of confidence, and at the other end of the range, a lowest minimum time period (fastest scrolling) may be applied to images that are determined to have the highest measure of confidence. Between these two ends, the minimum time period may decrease as confidence increases, in a number of adjacent bands. For example, a minimum time period and corresponding scrolling speed may be defined in accordance with a particular confidence interval, for example as in the following table:

| **Minimum time period** | **Scrolling speed** | **Measure of confidence** |
|---|---|---|
| **Longest** | Normal "slow" scrolling | Low (0%) - medium (60%) confidence levels for that slice |
| **Long** | Enhanced scrolling | 60% - 80% confidence levels for that slice |
| **Medium** | Accelerated scrolling | 80% - 90% confidence levels for that slice |
| **Short** | Hasted scrolling | 90% - 95% confidence levels for that slice |
| **Shortest** | Fast scrolling (just readable) | 95% - 100% confidence levels for that slice |

A time period associated with each band may then be defined according to need and human factors considerations.

In a variation to the method 20, the third step 230 comprises defining a minimum time period according to whether an AI finding has been made and also according to the confidence level.

In a variation to the method 20, the third step 230 comprises adapting the minimum time period according to the to a review history of the image, i.e., according to whether or not an AI determination in relation to the image has been reviewed previously. This could influence the significance of reviewing it thoroughly again, and therefore the scrolling speed can be adjusted. In an example method, the system measures one or more of whether or not: the radiologist slowed down / stopped scrolling through the anatomy around the finding (by analyzing computer behavior); the radiologist has looked at and around the finding (by analyzing eye-gaze data); and the radiologist has reported on that finding (by analyzing the report). The minimum time period is accordingly adapted.

In a variation to the method 20, the third step 230 comprises adapting the minimum time period according to a patient history or a finding history, or both. If this specific finding was reviewed and reported as insignificant before, and nothing has changed about the dimensions, positioning or orientation, it could be less significant to review it again. Therefore, as an example, in this scenario, the scrolling speed can be adjusted to be faster. Optionally, as an example, in this variation the method, one or more of visual, haptic, or auditive feedback is provided to indicate to the operator why this is happening.

In a variation to the method 20, the third step 230 comprises adapting the minimum time period according to an interpreted readability of finding. In this variation to the method, the interpreted readability of a finding is taken into account. For example, some findings can be interpreted more easily than others (e.g., due to similarities in tissue around the finding). This parameter can thus be used to adapt the minimum time period (and therefore the scrolling speed).

In a variation to the method 20, the third step 230 comprises adapting the minimum time period according to a trust level in the AI. Early after the implementation of such an application, the scrolling speed may be reduced significantly, since trust may be lower in the accuracy of the AI, and more time may be required to review AI findings. At a later stage, when the user has sufficient trust in the AI's performance, the overall scrolling speeds can be adapted to a higher (nuanced) value. A trust level may be manually adjusted by an operator, which may then be used to adapt the minimum time period.

In a variation to the method 20, the third step 230 comprises adapting the minimum time period according to a functionality determination. In specific situations it is possible that a radiologist may not wish to use the adaptive scrolling support. This variation on the method comprises an engine that controls the enablement of step 230, based on the context of use. For example, it may be beneficial not to enable this system during educational processes, consultations, and more. This embodiment facilitates the autonomous switching for a lower chance of disruptions and confusion, and an overall improved user experience. In an alternative variation, the method may omit the third step 230, according to an operator input. In other words, the operator may manually over-ride the determination of a minimum time period, i.e., may manually disable the adaptive scrolling.

In a variation to the method 20, the fourth step 240 comprises provision of haptic feedback according to minimum time period (and therefore according to scrolling speed). If the slice scrolling is slowed down, the operator could be provided with increased physical feedback in terms of resistance. By providing additional haptic feedback to the operator, operators can be made even more conscious about the necessary attentiveness during that moment in the review.

In an variation to the method 20: the second step 220 comprises processing the set of medical images using at least one machine-learning method, to determine or predict, for each medical image, a predetermined pathology characteristic; and the third step 230 comprises defining, for each medical image in the set of medical images, a minimum time period for which the medical image is to be displayed, when displayed at the user interface, responsive to the predetermined pathology characteristic. The predetermined pathology characteristic may comprise one or more of: a level of confidence that said medical image contains a representation of one or more predetermined pathologies; the nature of the pathologies; the size and/or number of the pathologies; the indication of the study; or any combination of the above.

The variations on the method set out herein may be combined individually with the method 20 or may combined in any combination within the method 20. For example, all variations on the method may be combined in one exemplary method according to the invention.

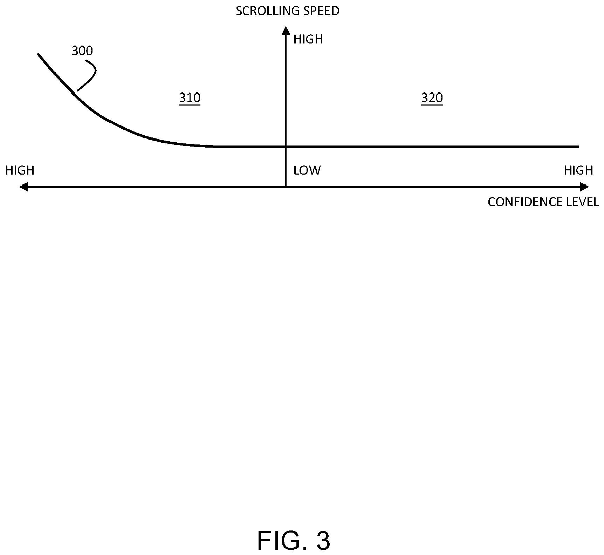

Fig 3 is a chart showing a variation of minimum time period / scrolling speed against confidence level, according to an embodiment. Along the horizontal axis is the confidence level, divided into two portions 310, 320. Left-hand portion 310 relates to where there is no AI finding and right-hand portion 320 relates to where there is an AI finding. The vertical axis defines the scrolling speed. A function 300 of scrolling speed according to confidence level is defined. A fast scrolling speed is not desirable when images may need to be more carefully reviewed by an operator. Considering the no AI finding left-hand portion 310 of Fig 3, function 300 allows the operator to scroll quickly when there is no AI finding but when confidence is high. Confidence may be high because the image has previously been reviewed by the operator. Function 300 however, limits the scrolling speed when there is an AI finding, i.e., when AI has determined that said medical image contains a representation of one or more predetermined pathologies, regardless of the confidence level (i.e., the measure of confidence). This allows the operator to carefully check the images to confirm the finding of the AI. Function 300 therefore defines a manner in which scrolling speed can be adapted according to a measure of confidence. This function may be well suited to an early adoption of the technology, where scrolling speed is automatically lowered any time there is an AI finding, regardless of confidence, so that each finding is carefully checked by an operator.

With continued reference to Fig. 3, a fastest scrolling speed maximizes scrolling efficiency but always maintains a certain level of readability, and thus may be limited according to a predetermined limit. This velocity can be calculated and tested through human perception studies. This way a tailored use of adaptive slice scrolling can be derived. Since the radiologist has the ultimate responsibility to check all the findings thoroughly, it is important when coming across a finding for the first time, the speed is lowered until it is fully reviewed.

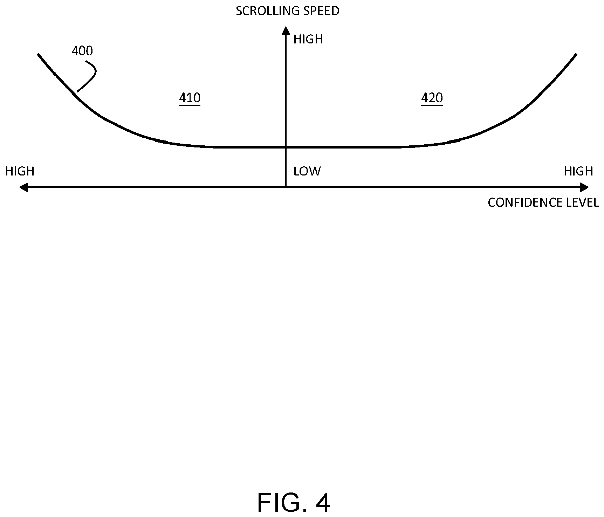

Fig 4 is a chart showing a variation of minimum time period / scrolling speed against confidence level, according to another embodiment, which may relate to a scenario where findings have been reviewed before. In this scenario, scrolling speed can be adapted when there is higher confidence in the identification of the AI. Again, along the horizontal axis is the confidence level, divided into two portions 410, 420. Again, left-hand portion 410 relates to where there is no AI finding and right-hand portion 420 relates to where there is an AI finding. Again, the vertical axis defines the scrolling speed. A function 400 of scrolling speed according to confidence level is defined. Here, again, the function 400 defines a faster scrolling speed where confidence is high in the left-hand no AI finding portion 410. In the right-hand AI finding portion 420, representative of when AI having determined that said medical image contains a representation of one or more predetermined pathologies, scrolling speed varies according to confidence. Where the confidence is higher, the scrolling speed is faster than when confidence is lower. Thus, the system may automatically display images for a shorter period where the AI measure of confidence is high, since a shorter review period may be required than where confidence is low. Function 400 therefore defines an alternative manner in which scrolling speed can be adapted according to a measure of confidence. Function 400 may suit a scenario where user confidence in the system is proven, and the operator has become confident that less time is required to review the AI findings with a higher level of confidence. Accordingly, the operator may spend more time reviewing images with a low level of confidence, and more time reviewing images with a low level of confidence. Accordingly, their cognitive load may be more focused, fatigue may be reduced and user confidence in the value of the system may be established more quickly (since unnecessary review of high confidence findings are reduced).

Additionally, in a futuristic scenario, AI performance, trustworthiness and adoption could possibly be so high that the ultimate responsibility shifts to AI analyses instead of the radiologist. In this case, the function 400 may be adapted so that scrolling speed is increased for all unreviewed AI findings, which may reflect a very high level of confidence in the system.

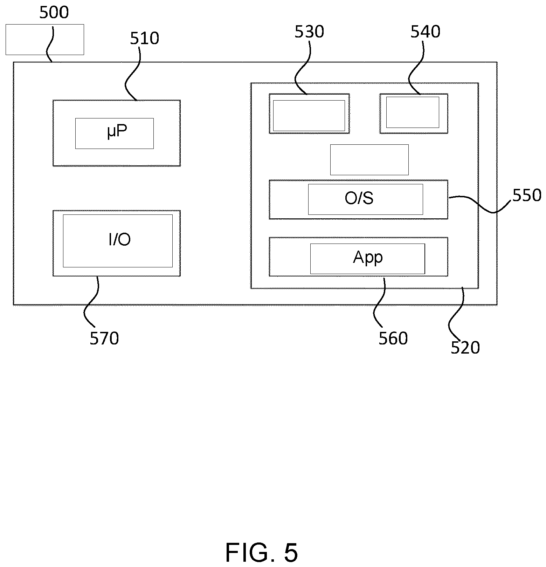

Fig 5 is a simplified block diagram of a computer within which one or more parts of an embodiment may be employed. Fig 5 illustrates an example of a computer 500 within which one or more parts of an embodiment may be employed. Various operations discussed above may utilize the capabilities of the computer 500. For example, one or more parts of a system for providing a subject-specific user interface may be incorporated in any element, module, application, and/or component discussed herein. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520, and one or more I/O devices 570 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor-based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 540, source code 530, and one or more applications 560 in accordance with exemplary embodiments. As illustrated, the application 560 comprises numerous functional components for implementing the features and operations of the exemplary embodiments. The application 560 of the computer 500 may represent various applications, computational units, logic, functional units, processes, operations, virtual entities, and/or modules in accordance with exemplary embodiments, but the application 560 is not meant to be a limitation.

The operating system 550 controls the execution of other computer programs, and provides scheduling, input-output control, file and data management, memory management, and communication control and related services. It is contemplated by the inventors that the application 560 for implementing exemplary embodiments may be applicable on all commercially available operating systems.

Application 560 may be a source program, executable program (object code), script, or any other entity comprising a set of instructions to be performed. When a source program, then the program is usually translated via a compiler (such as the compiler 540), assembler, interpreter, or the like, which may or may not be included within the memory 520, so as to operate properly in connection with the O/S 550. Furthermore, the application 560 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 570 may include input devices such as, for example but not limited to, a mouse, keyboard, scanner, microphone, camera, etc. Furthermore, the I/O devices 570 may also include output devices, for example but not limited to a printer, display, etc. Finally, the I/O devices 570 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 570 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at startup, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 560 and the O/S 550 are read, in whole or in part, by the processor 510, perhaps buffered within the processor 510, and then executed.

When the application 560 is implemented in software it should be noted that the application 560 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 560 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

A single processor or other unit may fulfil the functions of several items recited in the claims.

A computer program may be stored/distributed on a suitable medium, such as an optical storage medium or a solid-state medium supplied together with or as part of other hardware, but may also be distributed in other forms, such as via the Internet or other wired or wireless telecommunication systems.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figs illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figs. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

Any reference signs in the claims should not be construed as limiting the scope.

## Claims

1. A computer-implemented method (20) for aiding the review of a set of one or more medical images (110) for display at a user interface (150) for viewing by a viewer of the user interface, the computer-implemented method comprising:
obtaining (210) the set of medical images (110);
processing (220) the set of medical images using at least one machine-learning method, to predict, for each medical image, a measure of confidence that said medical image contains a representation of one or more predetermined pathologies;
defining (230), for each medical image in the set of medical images, a minimum time period for which the medical image is to be displayed, when displayed at the user interface, responsive to the measure of confidence for said medical image; and
displaying (240) at least one of the medical images (140) in the set of medical images at the user interface (150), wherein, for each medical image displayed, the time period for which said medical image is displayed is no less than the defined minimum time period for that medical image.

2. The computer-implemented method of claim 1, wherein, for each medical image, the minimum time period is configured to increase responsive to a measure of confidence for that medical image decreasing.

3. The computer-implemented method of claim 1 or 2, wherein:
the step of processing the set of medical images using the at least one machine-learning method further produces, for each medical image, a pathology indicator that indicates a prediction of whether or not said medical image contains the representation of the one or more predetermined pathologies; and
the step of defining, for each medical image, the minimum time period is further responsive to the pathology indicator for said medical image.

4. The computer-implemented method of claim 3, wherein, for each medical image, the minimum time period is configured to increase responsive to the pathology indicator for said medical image indicating that said medical image is predicted to contain a representation of the one or more predetermined pathologies.

5. The computer-implemented method of any of claims 1 to 4, wherein the step of displaying at least one of the medical images in the set comprises:
displaying, via the user interface, a first medical image of the set of medical images; and
responsive to receiving a scroll input from an input user interface, moving to a neighboring medical image, to the first medical image, in the set of medical images only if and/or when the first medical image has been displayed, via the user interface, for at least the minimum time period for the first medical image.

6. The computer-implemented method of any of claims 1 to 5, wherein the step of defining, for each medical image, the minimum time period is further responsive to the measure of confidence of at least one neighboring medical image, in the set of medical images, to the said medical image.

7. The computer-implemented method of any of claims 1 to 6, further comprising:
obtaining, for each medical image, a review indicator that indicates whether or not the medical image, or any potential pathology in the medical image, has been previously reviewed by a clinician,
wherein, for each medical image, the minimum time period for said medical image is further responsive to the review indicator of the medical image.

8. The computer-implemented method of claim 7, wherein, for each medical image, the minimum time period decreases responsive to the review indicator indicating that the medical image, or any potential pathology in the medical image, has been previously reviewed by a clinician.

9. The computer-implemented method of any of claims 1 to 8, further comprising obtaining a measure of concentration of the viewer of the user interface,
wherein, for each medical image, the minimum time period for said medical image is further responsive to the measure of concentration.

10. The computer-implemented method of claim 9, wherein, for each medical image, the minimum time period is configured to increase responsive to the measure of concentration decreasing.

11. The computer-implemented method of any of claims 1 to 10, further comprising obtaining an experience level of the viewer of the user interface,
wherein, for each medical image, the minimum time period for said medical image is further responsive to the experience level.

12. The computer-implemented method of claim 11, wherein, for each medical image, the minimum time period is configured to decrease responsive to the experience level increasing.

13. The computer-implemented method of any of claims 1 to 12, further comprising, whilst displaying the at least one of the medical images, providing haptic feedback to the viewer of the user interface responsive to the minimum time of the displayed medical image.

14. A computer program product comprising computer program code means which, when executed on a computing device having a processing system, cause the processing system to perform all of the steps of the method according to any of claims 1 to 13.

15. A processing system (10) for aiding the review of a set of one or more medical images (110) for display at a user interface (150) for viewing by a viewer of the user interface, the processing system being configured to:
obtain the set of medical images;
process the set of medical images using at least one machine-learning method, to predict, for each medical image, a measure of confidence that said medical image contains a representation of one or more predetermined pathologies;
define, for each medical image in the set of medical images, a minimum time period for which the medical image is to be displayed, when displayed at the user interface, responsive to the measure of confidence for said medical image; and
display at least one of the medical images (140) in the set of medical images at the user interface (150), wherein, for each medical image displayed, the time period for which said medical image is displayed is no less than the defined minimum time period for that medical image.
